# EUROPEAN PATENT APPLICATION

(11) **EP 1 553 165 A1**
(43) Date of publication of application: **13.07.2005**
(21) Application number: 05000232.8
(22) Date of filing: 07.01.2005
(51) Int. Cl.: C12M 1/00, C12M 3/00

(54) **Vessel for cell culture**

(30) Priority: 09.01.2004 JP 2004003778
(71) Applicant: Nipro Corporation, Osaka-shi, Osaka 531-8510 (JP)
(72) Inventor: Shirasu, Akio, Osaka-shi, Osaka 531-8510 (JP); Yoshikawa, Yoshihiro, Osaka-shi, Osaka 531-8510 (JP); Wada, Seiichi, Osaka-shi, Osaka 531-8510 (JP); Nakatani, Naomi, Osaka-shi, Osaka 531-8510 (JP)
(74) Representative: Albrecht, Thomas, Dr.

(57) **Abstract**

A vessel for cell culture includes a molded polymer sheet made of a polymer constituted by carbon atoms and hydrogen atoms attached to the carbon atoms, in which a portion of the hydrogen atoms are substituted by fluorine atoms. The vessel has more excellent cell growth ability than conventional vessels for cell culture, and hence enables more efficient cell culture.

## Description

### Technical Field of the Invention

The present invention relates to a plastic vessel for cell culture made of a polymer constituted by carbon atoms and hydrogen atoms attached to the carbon atoms, in which a part of the hydrogen atoms are substituted by fluorine atoms.

### Background of the Invention

In recent years, a field of regeneration medicine has been developed for the purpose of regenerating tissues and organs of a living body that suffer from functional disorder or dysfunction by positively utilizing cells. A technology involving extracting normal cells from a mammal, culturing the cells outside the living body, and then returning them to the living body forms a part of this field.

Conventionally, culture of attached cells or suspended cells of a mammal outside the living body has been carried out in a dish made of polystyrene. However, since such a vessel has low gas permeability, sufficient cell growth has not been obtained unless the thickness of the culture medium is set to about 3 mm and an air layer is provided in the vessel in order to allow sufficient exchange of oxygen, which is necessary for cell growth, or to allow carbon dioxide to be made. Therefore, when a large amount of cells are cultured, there is wasted space, thus requiring much space.

To solve such a problem, there have been reported among others a vessel for cell culture formed from an ionomer (JP Sho 60-160881 A; EP 148161 A) , a vessel for cell culture formed from a polyethylene sheet (Japanese Utility Model Unexamined Publication No.1-99200; JP 2-255077 A; JP 3-172169 A), a vessel for cell culture formed from a poly-4-methylpentene-1 resin and a polyolefin resin other than poly-4-methylpentene-1 resin (JP 2001-190267 A), and a cultivation apparatus in which at least a part thereof including the whole apparatus is constituted of a fluorine-containing meltable resin (JP 63-198972 A; Japanese Utility Model Unexamined Publication No. 62-68599). These vessels for cell culture each have high gas permeability so that they allow culturing to be carried out with a culture medium fully filled in the vessels. Therefore, the culturing can be performed in a much smaller space in them than in a dish made of polystyrene so that the vessels are suitable for large-scale culturing.

However, to further reduce the culturing space, a vessel for cell culture that has an excellent cell growth effect and is suitable for large-scale culturing has been needed.

### Summary of the Invention

Development of a vessel for cell culture that prevents contamination of foreign matter into the medium, has higher ability of cell growth, and is more suitable for large-scale culturing than conventional vessels for cell culture has been demanded.

The present invention relates to:
(1) a vessel for cell culture molded from a polymer sheet, wherein hydrogen atoms attached to the carbon atoms constituting the polymer are partly substituted by fluorine atoms;
(2) the vessel for cell culture according to item (1), wherein hydrogen atoms are partly substituted by fluorine atoms at a surface of the vessel molded from the polymer sheet;
(3) the vessel for cell culture according to item (1), wherein hydrogen atoms are partly substituted by fluorine atoms at an inner surface of the vessel molded from the polymer sheet;
(4) the vessel for cell culture according to item (1), wherein the polymer is polyvinyl chloride, polystyrene, a polyester, a silicone-based polymer or a polyolefin;
(5) the vessel for cell culture according to item (1), wherein the polymer is polyethylene;
(6) the vessel for cell culture according to item (1), wherein an oxygen permeability coefficient of the vessel is about 100 to 5,000 cm³/m² · 24hr · atm;
(7) the vessel for cell culture according to item (1), wherein a carbon dioxide permeability coefficient of the vessel is about 1,000 to 20,000 cm³/m² · 24hr · atm;
(8) a vessel for cell culture molded from a polymer sheet, wherein hydrogen atoms attached to the carbon atoms constituting the polymer are partly substituted by fluorine atoms, obtained by reacting a vessel molded from the polymer sheet with a fluorine gas in the presence of an inert gas;
(9) the vessel for cell culture according to item (8), wherein a content of the fluorine gas is about 0.1 to 20.0 vol.% relative to the inert gas;
(10) the vessel for cell culture according to item (8), wherein an inner surface of the vessel molded from the polymer sheet is reacted with a fluorine gas in the presence of an inert gas;
(11) a method of producing a vessel for cell culture molded from a polymer sheet, wherein hydrogen atoms attached to the carbon atoms constituting the polymer are partly substituted by fluorine atoms, which comprises reacting a vessel molded from the polymer sheet with a fluorine gas in the presence of an inert gas; and
(12) the method of producing a vessel for cell culture according to item (11), wherein an inner surface of the vessel molded from the polymer sheet is reacted with a fluorine gas in the presence of an inert gas.

### Effect of the Invention

The vessel for cell culture according to the present invention prevents contamination of foreign matter into the medium, has higher ability of cell growth, and is more suitable for large-scale culturing than conventional vessels for cell culture.

### Detailed Description of the Invention

The vessel for cell culture according to the present invention is a plastic vessel for culturing cells, which is characterized in that hydrogen atoms attached to carbon atoms that constitute the polymer are partly substituted by, or with fluorine atoms (hereinafter, also referred to as "fluorine-substituted").

The term "fluorine-substituted" means that a portion of the hydrogen atoms attached to carbon atoms that constitute the polymer are substituted with fluorine atoms. The term "a portion of" herein means that assuming that the proportion in number of hydrogen atoms attached to carbon atoms that constitute the polymer before they are substituted with fluorine atoms is 100%, the proportion in number of hydrogen atoms substituted by fluorine atoms is about 0.1 to 99.9%, preferably about 0.5 to 50%, more preferably about 1 to 10%, most preferably about 2 to 5%.

The shape of the polymer vessel of the present invention is not particularly limited but examples of the shape include bag and bottle shapes. However, a bag-shaped vessel formed from a flexible sheet that is readily mass-produced and is light in weight is preferable. Further, it is preferable that the vessel be provided with ports for letting a cell suspension in and out of the vessel, respectively.

Further, the polymer is not particularly limited so far as a part of hydrogen atoms attached to carbon atoms that constitute the polymer can be substituted with fluorine atoms by fluorine gas. Specific examples of the polymer include thermoplastic resins such as polyvinyl chloride, polystyrene, polyesters, silicone-based polymers, and polyolefins. Of these, a polyolefin is preferred. Specific examples of polyolefins include polyethylene, polypropylene, polystyrene, and poly-4-methylpentene. Of those, polyethylene is preferred.

Further, in the vessel for cell culture according to the present invention, fluorine substitution may be made on the inner surface or outer surface, or both surfaces of the polymer vessel. It is preferable that the inner surface of the vessel be fluorine-substituted.

The vessel for cell culture according to the present invention preferably has sufficient breathability for culturing cells. Specifically, the vessel has: an oxygen permeability coefficient of about 100 to 5,000 cm³/m² · 24hr · atm, preferably about 1,100 to 3, 000 cm³/m² · 24hr · atm, more preferably about 1,250 to 2, 750 cm³/m² · 24hr · atm; and a carbon dioxide permeability coefficient of about 1,000 to 20,000 cm³/m² · 24hr · atm, preferably about 3, 000 to 11, 500 cm³/m² · 24hr · atm, more preferably about 5, 000 to 9,000 cm³/m² · 24hr · atm.

In the present invention, the fluorine substitution is achieved by reacting the inner and/or the outer vessel molded from a polymer sheet with a fluorine-containing gas in the presence of an inert gas. However, the method is not limited to this so far as a similar effect is obtained. The inert gas may be any gas that does not inhibit the reaction at the time of fluorine substitution, such as nitrogen or argon. The content of the fluorine gas with respect to the inert gas is about 0.1 to 20.0 vol%, preferably about 0.1 to 10 vol%. The reaction temperature is about - 20 to 150°C, preferably about 0 to 40°C. The higher the temperature, the higher the reaction rate of fluorine substitution. However, care must be taken to avoid a disaster such as fire. Further, the pressure condition is about 0.01 to 10.0 atm, preferably about 0.01 to 2.0 atm, more preferably about atmospheric pressure. The reaction time is about 30 seconds to 200 minutes, preferably about 1 to 200 minutes, more preferably about 1 to 20 minutes, when the pressure condition is near atmospheric pressure.

The vessel for cell culture according to the present invention can be produced by means of a method involving performing fluorine substitution on a molded polymer vessel or a method involving molding a polymer that has been previously fluorine-substituted into a vessel. Here, the production of the polymer vessel can be practiced using a plastic sheet by means of a conventional method. The thickness of the plastic sheet is preferably about 50 to 300 µm. For example, when the polymer vessel is in the form of a bag, the polymer vessel is produced by: preparing a flexible sheet by means of an inflation method, a T-die method, or the like; and molding the flexible sheet by means of a heat-sealing method.

The method of directly performing fluorine substitution on a molded polymer vessel involves: providing an inlet port and an outlet port for a cell suspension in a sheet prepared from a polymer by means of an inflation method or T-die method; molding the sheet into a bag; and flowing an inert gas containing fluorine through the inlet port and outlet port inside the bag to prepare the vessel for cell culture. The inflation method may vary depending on the kind of polymer. For example, when polyethylene is used, the vessel can be prepared at a melting temperature of about 160 to 180°C and a take-up speed of about 10 to 30 m/min. As for the conditions of fluorine substitution, the vessel can be prepared by performing the fluorine substitution by using an inert gas containing about 0.1 to 20.0 vol%, preferably about 0.1 to 10 vol%, of fluorine gas therein at about 25°C and under atmospheric pressure for about 30 seconds to 200 minutes, preferably about 1 to 200 minutes, more preferably about 1 to 20 minutes.

The method of molding the previously fluorine-substituted polymer into a vessel includes a method involving preparing a polymer sheet, only one side of which is fluorine-substituted, and molding the sheet into a bag in a state where the sheet is provided with ports. Specifically, the bag is prepared by means of the method described in JP 62-140821 A or a method similar thereto. When the polymer is molded into a sheet by means of an inflation method, the fluorine substitution is performed by flowing an inert gas containing about 0.1 to 20.0 vol%, preferably about 0.1 to 10 vol% of fluorine gas into the inside of the bag. The conditions in the inflation method may vary depending on the kind of polymer. For example, when polyethylene is used, the sheet can be prepared at a melting temperature of about 160 to 180°C and a take-up speed of about 10 to 30 m/min. The vessel can be prepared by molding the thus-obtained sheet into a bag by means of a heat-sealing method at a temperature condition of about 170 to 190°C.

Further, the above-mentioned substitution reaction by fluorine can be performed more effectively by carrying it out in combination with other treatments such as a plasma treatment and a corona discharge treatment. For example, in the case of corona discharge, the reaction is performed at a voltage of about 10 to 30 kV, preferably about 15 to 25 kV, and a frequency of about 10 to 30 kHz, preferably about 15 to 25 kHz. In addition, heating at a temperature lower than the melting point of the material by about 20 to 130°C is more preferable. The heating methods include irradiation with infrared radiation and hot air blowing, but are not particularly limited.

The method of culturing cells by using the vessel for cell culture obtained by means of the above-mentioned production method can be performed according to a conventional method. For example, the cells to be cultured include floating cells such as umbilical cord blood cells, hematopoietic stem cells, lymphocytes and hybridomas, and internal organs forming parenchymal cells such as hepatic cells. Further, the media to be used may be commercially available basic media such as Eagle MEM medium, DMEMmedium, RPMI1640 medium, HamF10 medium, and HamF12 medium. The concentration of inoculated cells is about 1.0 × 10⁴ to 5.0 × 10⁵ cells/ml, preferably about 1.0 × 10⁵ to 2. 0 × 10⁵ cells/ml. The method enables high density culture and mass production of cells.

Hereinafter, the present invention will be described by way of examples and test examples.

### Example 1

Linear low-density polyethylene (available from Mitsui Chemical Corporation, Japan) was formed into a sheet (thickness: 100µm) by means of a T-die method. The conditions of the T-die method were a melting temperature of 170°C and a take-up speed of 25 m/min. The sheet was cut into pieces of 6 × 10 cm square. Two of the obtained polyethylene sheets were laminated in a state where inlet and outlet ports for a cell suspension were provided for the sheets, and ends of the sheets were heat-sealed to mold the sheets into a bag shape to prepare a polyethylene bag as a vessel for cell culture. A nitrogen gas containing 10 vol% of fluorine gas was flowed into the inside of the bag through the inlet and outlet ports of the vessel for cell culture under atmospheric pressure and at 30°C for 1 minute. Thus, a vessel for cell culture was prepared.

### Example 2

Linear low-density polyethylene (available from Mitsui Chemical Corporation) was formed into a sheet (thickness: 100µm) by means of a T-die method. The conditions of the T-die method were a melting temperature of 170°C and a take-up speed of 25 m/min. The sheet was cut into pieces of 6 × 10 cm square. Two of the obtained polyethylene sheets were laminated in a state where inlet and outlet ports for a cell suspension were provided for the sheets, and ends of the sheets were heat-sealed to mold the sheets into a bag shape to prepare a polyethylene bag as a vessel for cell culture. A nitrogen gas containing 10 vol% of fluorine gas was flowed into the inside of the bag through the inlet and outlet ports of the vessel for cell culture under atmospheric pressure and at 30°C for 1 or 180 minutes. Thus, two kinds of vessels for cell culture were prepared.

### Example 3

Linear low-density polyethylene (available from Mitsui Chemical Corporation) was formed into a sheet (thickness: 100µm) by means of an inflation method. The conditions of the inflation method were a melting temperature of 170°C and a take-up speed of 25 m/min. The sheet was cut into pieces of 10 cm square. Two of the obtained polyethylene sheets were laminated in a state where inlet and outlet ports for a cell suspension were provided for the sheets, and ends of the sheets were heat-sealed to mold the sheets into a bag shape to prepare a polyethylene bag as a vessel for cell culture. Anitrogen gas containing 15 vol% of fluorine gas was flowed into the inside of the bag through the inlet and outlet ports of the vessel for cell culture under atmospheric pressure and at 25°C for 1 minute. Thus, a vessel for cell culture was prepared.

### Example 4

Linear low-density polyethylene (available from Mitsui Chemical Corporation) was formed into a sheet (thickness: 100µm) by means of an inflation method. The conditions of the inflation method were a melting temperature of 170°C and a take-up speed of 25 m/min. The sheet was cut into pieces of 10 cm square. Two of the obtained polyethylene sheets were laminated in a state where inlet and outlet ports for a cell suspension were provided for the sheets, and ends of the sheets were heat-sealed to mold the sheets into a bag shape to prepare a polyethylene bag as a vessel for cell culture. A nitrogen gas containing 5 vol% of fluorine gas was flowed into the inside of the bag through the inlet and outlet ports of the vessel for cell culture under atmospheric pressure and at 25°C for 5 minutes. Thus, a vessel for cell culture was prepared.

### Example 5

Linear low-density polyethylene (available from Mitsui Chemical Corporation) was formed into a sheet (thickness: 100µm) by means of an inflation method. The conditions of the inflation method were a melting temperature of 170°C and a take-up speed of 25 m/min. The sheet was cut into pieces of 10 cm square. Two of the obtained polyethylene sheets were laminated in a state where inlet and outlet ports for a cell suspension were provided for the sheets, and ends of the sheets were heat-sealed to mold the sheets into a bag shape to prepare a polyethylene bag as a vessel for cell culture. A nitrogen gas containing 10 vol% of fluorine gas was flowed into the inside of the bag through the inlet and outlet ports of the vessel for cell culture under atmospheric pressure and at 25°C for 10 minutes. Thus, a vessel for cell culture was prepared.

### Test Example 1 Elemental Analysis of inner surface of the vessel (bag) and Gas Permeability

For the vessel for cell culture of Example 1, and , as a comparison, a vessel ① made of polyethylene (PE) before reacting with fluorine as described in Example 1, a vessel ② made of polytetrafuluoroethylene(PTFE, thickness:100µm) without reacting with fluorine, and a vessel ③ made of copolymer of tetrafluoroethylene-hexafluoropropylene (FEP, thickness:100µm)) without reacting with fluorine, an elemental analysis of the inner surface of these four vessels was conducted, and the gas permeability of these vessels was measured.

The elemental analysis was conducted by fluorescent X-ray method using an X-ray fluorescence Spectrometer (XRF-1700, Shimazu Corporation, Japan).

The measurement of permeability of oxygen gas and carbon dioxide gas was based on Japanese Industrial Standard JIS K 7126, (ISO 2556) and was measured by using a permeability measuring device (MT-C3, Toyoseiki Seisaku-sho, Ltd., Japan).

The results are shown in Table 1.

**Table 1**

| Elemental analysis of the inner surface of vessel and gas permeability | | | | |
|---|---|---|---|---|
| Test Sample | Elemental Analysis | | Gas Permeability (cm³/m² · 24hr · atm) | |
| | C (%) | F (%) | O₂ | CO₂ |
| Ex. 1 | 93.3 | 6.7 | 2565 | 10629 |
| ① | 100 | 0 | 3028 | 11676 |
| ② | 14.8 | 85.2 | - | - |
| ③ | - | - | 2056 | 4431 |

Gas permeability of the vessel of Example 1 was almost the same as that of vessel ① as a comparison. Oxygen permeability of the vessel ③ as a comparison was about 80 % of that of the vessel of Example 1, and carbon dioxide permeability was about 40 % of that of the vessel of Example 1.

Fluorine atoms were not detectable on the outer surface of the vessel (bag shape) of Example 1, and this result suggests that very shallow parts of the inner surface were substituted by fluorine atoms

### Test Example 2 Cell Proliferation

In the vessel for cell culture of Example 1 and the vessel made of polyethylene before reacting with fluorine as described in Example 1 (comparison ①), respectively, 5 ml of RPMI1640 medium containing 10% fetal calf serum having a cell strain of human leukemia, MOLT-4 cells, suspended therein (Seeding concentration of MOLT-4 cells: 1.0 × 10⁴ cells/ml) were added and cultured for 10 days at 37°C/5%CO₂/99%RH (Relative Humidity) . After the culturing, a portion of the culture was sampled and the cell concentration in the vessel (bag) was determined with a hemocytometer.

The results are shown in Table 2.

**Table 2**

| Concentration of MOLT-4 cells (× 10⁴ cells/ml) | | | | | | |
|---|---|---|---|---|---|---|
| Test sample | Terms for culture (days) | | | | | |
| | 5 | 6 | 7 | 8 | 9 | 10 |
| Example 1 | 16 | 32 | 60 | 101 | 164 | 191.8 |
| Comparison ① | 10.5 | 19 | 33.3 | 60.5 | 72.5 | 116.5 |

The vessel of Example 1 shows more excellent cell proliferation ability than the comparison ①.

### Test Example 3 Cell Proliferation

In the two kinds of vessels for cell culture in Example 2, i. e. a vessel for cell culture treated with fluorine gas for 1 minute (the present invention ①) and a vessel for cell culture treated with fluorine gas for 180 minutes (the present invention ②), and a flask for cell culture made of polystyrene (25cm² flask, Catalog No.430639, Corning Co., U.S.A) as a comparison, 5 ml of RPMI1640 medium containing 10% fetal calf serum having a cell strain of human leukemia, MOLT-4 cells, suspended therein (Seeding concentration of MOLT-4 cells: 1.0 × 10⁴ cells/ml) were added and cultured for 6 days at 37°C/5%CO₂/99%RH. Six days after the culturing, a portion of the culture was sampled and the cell concentration in the bag was measured according to the method as described in Test Example 2.

The cell concentration is shown in Table 3

**Table 3**

| Concentration of MOLT-4 cells (× 10⁵ cells/ml) | | |
|---|---|---|
| Test sample | Before culturing | 6 days after Culturing |
| The present Invention ① | 1.0 | 25.4 |
| The present Invention ② | 1.0 | 22.5 |
| Comparison | 1.0 | 20.5 |

The vessel of the present invention ① shows more excellent cell proliferation ability than the vessel of the comparison.

### Test Example 4 Elemental analysis of inner surface of the vessel and contact angle of water

For the two kinds of vessels for cell culture obtained in Example 2, i.e. a vessel for cell culture treated with fluorine gas for 1 minute (the present invention ①) and a vessel for cell culture treated with fluorine gas for 180 minutes (the present invention ②), and a flask for cell culture made of polystyrene (25cm² flask, Catalog No. 430639, Corning Co., U. S.A) as a comparison, an elemental analysis (carbon and fluorine atoms) of the inner surface of the plastic film was conducted and the contact angle of water was measured. The results are shown in Table 4.

The elemental analysis was conducted according to the method as described in Test Example 1, and the contact angle of water to plastic film was measured according to the method of JIS K 6768 (ISO 8296).

**Table 4**

| Elemental Analysis and Contact Angle of Water | | | |
|---|---|---|---|
| Test sample | Elemental Analysis | | Contact Angle |
| | C (%) | F (%) | of water ( ° ) |
| The present Invention ① | 93.3 | 6.7 | 76 |
| The present Invention ② | 35.6 | 64.4 | 94 |
| Comparison | - | - | 56.8 |

It is apparent from data of the contact angle of water that the inner surface of the vessel of the present invention ① shows a slight hydrophilic property.

## Claims

1. A vessel for cell culture molded from a polymer sheet, wherein hydrogen atoms attached to carbon atoms constituting the polymer sheet are partly substituted by fluorine atoms.

2. The vessel for cell culture according to claim 1, wherein hydrogen atoms are partly substituted by fluorine atoms at a surface of said vessel molded from the polymer sheet.

3. The vessel for cell culture according to claim 1, wherein hydrogen atoms are partly substituted by fluorine atoms at an inner surface of said vessel molded from the polymer sheet.

4. The vessel for cell culture according to claim 1, wherein the polymer is polyvinyl chloride, polystyrene, a polyester, a silicon-based polymer or a polyolefin.

5. The vessel for cell culture according to claim 1, wherein the polymer is polyethylene.

6. The vessel for cell culture according to claim 1, wherein an oxygen permeability coefficient of the vessel is about 100 to 5, 000 cm³/m² · 24hr · atm.

7. The vessel for cell culture according to claim 1, wherein a carbon dioxide permeability coefficient of the vessel is about 1, 000 to 20,000 cm³/m² · 24hr · atm.

8. A vessel for cell culture molded from a polymer sheet, wherein hydrogen atoms attached to carbon atoms constituting the polymer sheet are partly substituted by fluorine atoms, and which is obtained by reacting a vessel molded from the polymer sheet with a fluorine gas in the presence of an inert gas.

9. The vessel for cell culture according to claim 8, wherein a content of the fluorine gas is about 0.1 to 20.0 vol% relative to the inert gas.

10. The vessel for cell culture according to claim 8, wherein an inner surface of the vessel molded from the polymer sheet is reacted with a fluorine gas in the presence of an inert gas.

11. A method of producing a vessel for cell culture molded from a polymer sheet, wherein hydrogen atoms attached to carbon atoms constituting the polymer sheet are partly substituted by fluorine atoms, which comprises reacting a vessel molded from the polymer sheet with a fluorine gas in the presence of an inert gas.

12. The method of producing a vessel for cell culture according to claim 11, wherein an inner surface of the vessel molded from the polymer sheet is reacted with a fluorine gas in the presence of an inert gas.
